# FASCICULE DE BREVET EUROPEEN

(11) **EP 0 647 623 B1**
(45) Date de publication et mention de la délivrance du brevet: **10.09.1997**
(21) Numéro de dépôt: 94402224.3
(22) Date de dépôt: 05.10.1994
(51) Int. Cl.: C07C 403/14, C07C 47/21, C07C 47/232, C07C 49/796, C07C 49/217, C07C 45/54, C07C 45/67

(54) **Nouveaux intermédiaires de préparation de la vitamine A et des caroténoides et leur procédé de préparation**
Zwischenprodukte zur Herstellung von Vitamin A und von Carotenoiden und Verfahren zu deren Herstellung
Intermediates for the preparation of vitamin A and carotenoids and process for their preparation

(30) Priorité: 07.10.1993 FR 9311943
(43) Date de publication de la demande: 12.04.1995
(73) Titulaire: RHONE-POULENC NUTRITION ANIMALE, 92160 Antony (FR)
(72) Inventeur: Bienayme, Hugues, F-69002 Lyon (FR)
(74) Mandataire: Le Pennec, Magali

(56) Documents cités:
- FR-A- 1 313 362
- US-A- 3 057 888
- CHEMICAL ABSTRACTS, vol. 103, no. 15, 14 Octobre 1985, Columbus, Ohio, US; abstract no. 123059d, 'Alpha - Beta - Unsaturated Carbonyl Compounds' page 688 ;colonne 2 ; & JP-A-60 032 742 (NIPPON ZEON CO., LTD.) 19 Février 1985

## Description

La présente invention concerne des nouveaux intermédiaires pour la préparation de la vitamine A et des caroténoïdes.

Elle concerne plus particulièrement des intermédiaires répondant à la fomule (I) suivante : 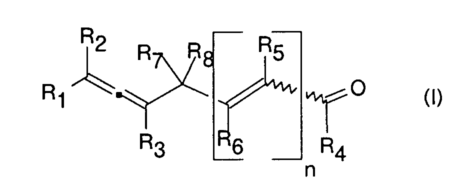 dans laquelle - n est égal à 1 ou 2
- R₁, R₂, R₃ identiques ou différents représentent chacun indépendamment l'un de l'autre l'hydrogène, un motif alkyle contenant 1 à 4 atomes de carbone, alkényle contenant 2 à 10 atomes de carbone, aryle, chaque motif alkyle et/ou alkényle pouvant être linéaire, ramifié ou cyclique, deux groupes peuvent former ensemble un motif alkylidène contenant de 2 à 12 atomes de carbone.
- R₄, R₅, R₆, R₇, R₈ identiques ou différents représentent chacun indépendemment l'un de l'autre l'hydrogène, un groupe alkyle contenant 1 à 4 atomes de carbone, alkenyle contenant 2 à 10 atomes de carbone, chaque motif alkyle, alkényle étant linéaire, ramifiée ou cyclique, aryle contenant 6 à 10 atomes de carbone ; deux groupes peuvent former ensemble un motif alkylidene contenant 2 à 10 atomes de carbone.

On préfère parmi les composés de formule (I) ceux pour lesquels R₄, R₅, R₇ et R₈ représentent l'hydrogène et R₆ un groupe méthyle.

On préfère tout particulièrement parmi les composés de formule (I) ceux pour lesquels R₁ représente le motif suivant : 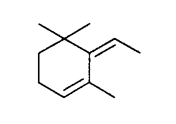 R₂ un groupe méthyle et R₃ l'hydrogène.

Le document FR 1 313 362 décrit la préparation d'aldéhyde porteur d'un motif allénique par réaction d'un alcool acétylénique avec un aldéhyde en présence d'un catalyseur acide ; cette réaction n'est pas applicable aux composés selon l'invention qui comporte des motifs vinyles.

Le document US 3 057 888 décrit la préparation d'aldéhyde porteur d'un motif allénique par hydrolyse d'un diester d'un aldéhyde allylique obtenu lui même à partir d'un ester acétylénique, ce type de réaction comme la précédente ne s'applique pas aux composés acétyléniques porteurs d'un motif vinyl.

Le document publié dans le Chemical Abstract Vol. 103, No. 15 abstract No. 113059d décrit la préparation de carbonates à partir de composés allyliques et de platine, ces carbonates sont ensuite traités avec du palladium pour former un aldéhyde allylique.

Aucun de ces documents ne décrit d'aldéhydes porteur d'un motif allénique et d'un motif vinylique.

Ces composés sont préparés par action d'un catalyseur à base d'un métal de transition sur un composé de fomule (II) suivante : 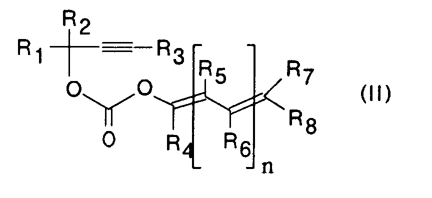 dans laquelle R₁, R₂, R₃, R₄, R₅, R₆, R₇ et R₈ et n ont la même signification que dans la formule (I).

On préfère tout particulièrement les composés de formule (II) suivants :

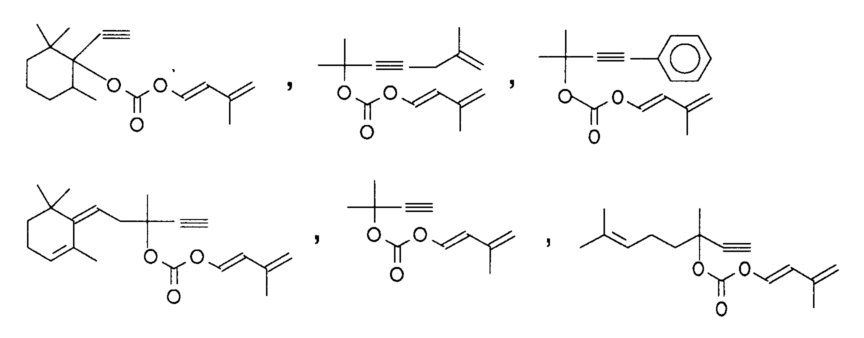

Le métal de transition est choisi notamment parmi les métaux des colonnes VIII, I_{B} et II_{B} de la classification périodique. On préfère utiliser le nickel ou le palladium.

Le métal de transition peut être utilisé en présence d'un ligand choisi parmi les les composés du phosphore telles que le bisdiphénylphosphinoéthane, la triphénylphosphine, la triméthoxyphénylphosphine, le triisopropylphosphite, la trifluorophénylphosphine, la trithiophènylphosphine, la tritolylphosphine, la trinaphtylphosphine. La réaction a lieu de préférence dans un solvant aprotique.

La quantité de catalyseur introduite varie avantageusement entre 1 et 20 % calculé en équivalent d'atome de métal par rapport au composé de formule (II).

Les composés de formule (I) sont avantageusement transformés en intermédiaires de la vitamine A ou d'aldéhydes terpéniques par action d'une base choisie parmi les carbonates, les alcoolates de métaux alcalins ou alcalinoterreux ou par action d'un acide tel que l'acide bromhydrique.

La présente invention sera plus complètement décrite à l'aide des exemples suivants qui ne doivent pas être considérés comme limitatifs de l'invention.

### EXEMPLE 1 : Carbonate de l'éthynyl rétroαionol et de méthyl-2 butadiène-1,3 ol-4

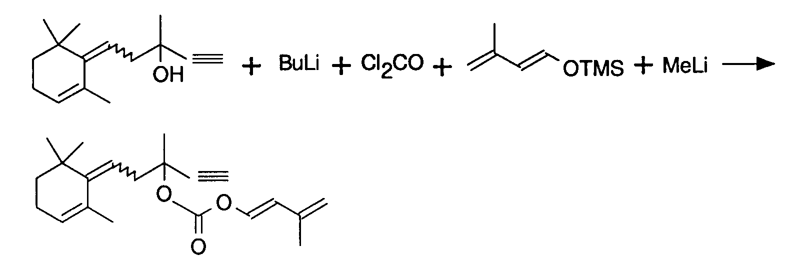

I) Dans 10 ml de THF anhydre contenant 1 g d'éthynyl rétroαionol (4,58 10-³ mole) sous argon, maintenu à -20°C, on ajoute 2,86 ml de butyllithium dans l'hexane (4,58 10⁻³ mole). A la fin de l'addition, on laisse reposer environ 10 minutes puis on ajoute à une solution de phosgène dans le toluène (2,36 ml, 4,57 mmole) à -40°C.
   A la fin de l'addition, la solution se colore en jaune orangé.
II) Dans 10 ml de THF contenant 6,46 10⁻³ mole d'éther d'énolsilylé du prénal, on ajoute à -40°C, 3,75 ml de méthyltithium (6 10⁻³ mole) en solution dans l'éther. On laisse 15 minutes puis on l'ajoute à -40°C, au chloroformiate préparé en I.
   On laisse 30 minutes à -40°C puis on dilue par l'éther éthylique, on lave avec une solution aqueuse de chlorure de sodium saturée. On réextrait à l'éther.
   On obtient après chromatographie 1,176 g (rendement 78%) de produit dont les analyses RMN infrarouge et de masse confirment la structure attendue.

### EXEMPLES 2 A 8

De la même manière qu'à l'exemple 1, on prépare selon la réaction suivante, les carbonates dont les rendements de préparation sont indiqués dans le tableau 1.

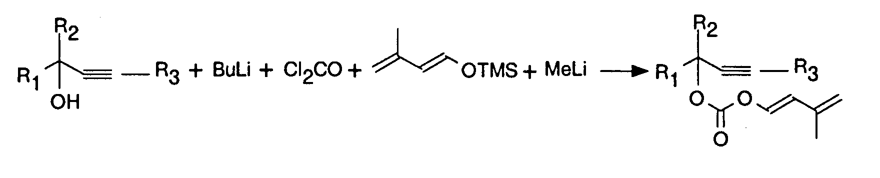

### EXEMPLE 9 (Tableau 1)

I) Préparation du chloroformiate de prényle 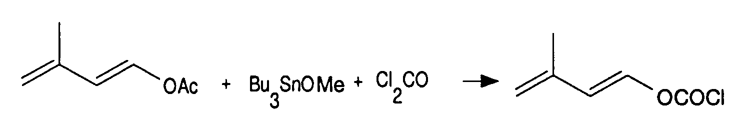
   On ajoute à de l'acétoxyisoprène fraîchement distillé (3,26g, 25,8 mmol) à 0°C, le méthoxytributylétain (7 ml, 24,3 mmol) sous argon. on agite à température ambiante pendant 30 minutes puis on dilue par 20 ml de chlorure de méthylène et on ajoute à 0°C à une solution toluénique de phosgène ( 12,5ml, 24mmol).
   On laisse sous agitation à 0°C pendant 15 minutes puis on évapore le chlorure de méthylène, on distille ensuite le toluène et le produit sous pression réduite (100°C, 90-100mm de mercure).
   On récupère 1,32g d'un liquide jaune clair contenant 74% en masse de chloroformiate attendu (RMN), le rendement est de 28%.
II) Préparation du carbonate 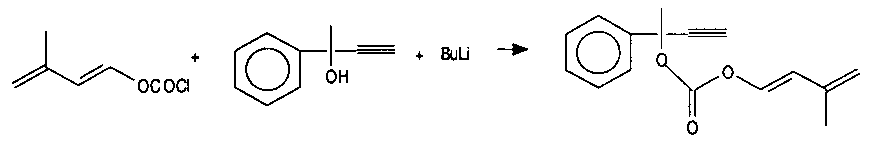
   Le 2-phényl 3-butyne 2-ol (0,664g, 4,54mmol) est dissout dans 5ml de tétrahydrofurane anhydre sous argon. On refroidit à -40°C et on ajoute le butyllithium (solution 1,6 molaire dans l'hexane, 3ml, 4,8mmol). On laisse sous agitation à - 40°C pendant 10 minutes puis on ajoute le chloroformiate préparé en I (1,20g, 6mmol) dans 1,5ml de tétrahydrofurane.
   Après 30 minutes à -40°C, on dilue par une solution de triéthylamine dans l'éther éthylique, on lave à l'eau à -20°C, on sèche sur sulfate de magnésium, on évapore sous pression réduite puis on chromatographie et on récupère le produit attendu (0,911g). Le rendement est de 78%.

### EXEMPLES 1 A 13 - REARRANGEMENT (Tableau 1 et 2)

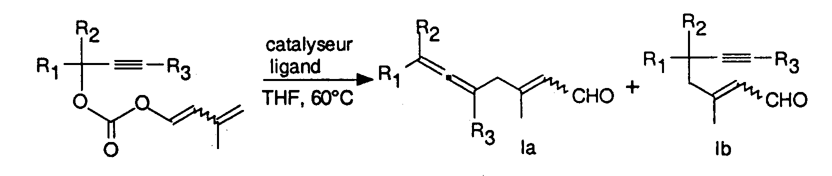

Le carbonate de l'exemple 2 R1=R2=CH₃ et R3=H (0,343g, 1,77 mmol) est dissous dans 6 ml de tétrahydrofurane anhydre sous argon. On ajoute à température ambiante le catalyseur (0,100 g, 0,086 mmol, 5 %) puis on porte à 65°C.

La réaction est suivie par chromatographie couche mince (éluant pentane/éther 10/1). Au bout de 1 heure 30 minutes, le carbonate a disparu. On évapore puis on chromatographie sur silice (éluant pentane/éther 10/1).

On sépare deux fractions :
- fraction a: masse 0,0568 g; produit Ia; rendement = 21 %
- fraction b: masse 0,1019 g; produit Ib; rendement = 38 %

Les exemples 1 à 9 sont réalisés selon le même mode opératoire que l'exemple 2, les résultats sont indiqués dans le tableau 1. Les exemples 10 à 13 sont réalisés selon le même mode opératoire que les exemples 1 à 9 avec différents catalyseurs et différents ligands, les résultats sont indiqués dans le tableau 2.

### EXEMPLES 14 A 18 : Préparation de différents carbonates selon la même procédure que celle de l'exemple 1.

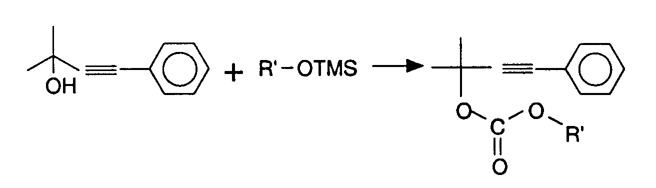

Le carbonate est réarrangé en présence d'une catalyse avec Pd(PPh₃)₄ 5 % dans du THF (Conc = 0,2) à 55-60°C.

Les composés obtenus ainsi que leur rendement respectif sont indiqués dans le tableau 3.

### EXEMPLE 19

On reproduit l'exemple 16 en utilisant comme catalyseur de réarrangement Ni(COD)₂ à 20 %, PPh₃ 40 % dans le THF à température ambiante pendant 3 heures. On obtient le composé suivant avec un rendement de 62 %.

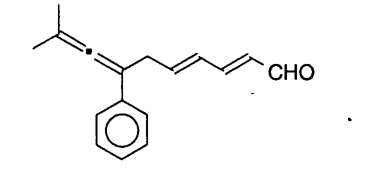

### EXEMPLES 20 : Utilisation d'un composé de formule (I) pour préparer un isomère du rétinal

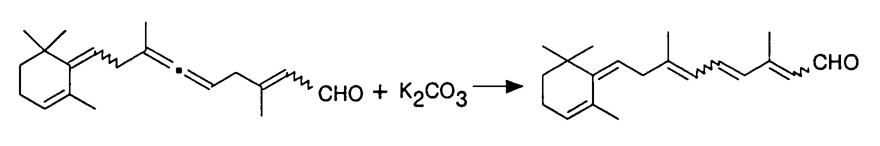

On introduit 0,069 g (0,244 10⁻³ mole) du dérivé obtenu à l'exemple 1 (Tableau I) (R₁ = 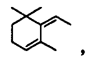 R₂ = CH₃, R₃ = H) dans 4 ml de méthanol, on ajoute sous argon, à température ambiante, le carbonate de potassium finement pulvérisé.

La solution se colore vite en orange. On laisse 25 minutes, on filtre sur colone de silice (éluant pentane/éther éthylique). On récupère 0,037 g d'un produit correspondant à la structure attendue dont le système polyénique n'est que partiellement reconjugué.

La rendement est de 54 %.

### EXEMPLE 21 : Utilisation d'un composé de formule (I) pour préparer le rétinal

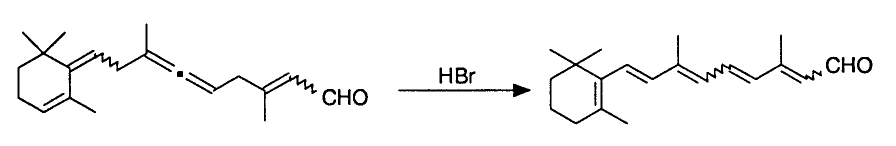

0,125 g (0,439 mmol) du composé obtenu à l'exemple 1 (Tableau 1) sont dissout dans 2 ml d'acétone. On refroidit à 0°C puis on ajoute 0,35 ml d'une solution d'acide bromhydrique dans l'acétone (c = 0,127). Au bout d'une heure, on traite par NaHCO₃. On dilue par Et₂O et on lave à l'eau.

La chromatographie sur silice donne 0,072 g de rétinal (57 %).

### EXEMPLE 22

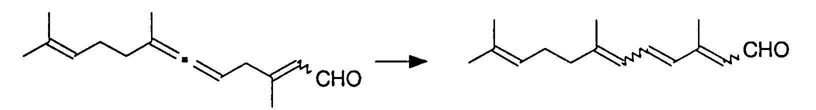

On reproduit le même mode opératoire qu à l'exemple 21 à partir du produit obtenu à l'exemple 8. Le produit obtenu est le déhydrofarnesal qui est obtenu avec un rendement de 75 %.

### EXEMPLE 23

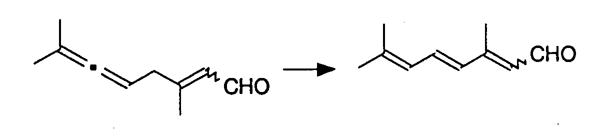

On reproduit le même mode opératoire que l'exemple 20 à partir du produit obtenu à l'exemple 2. Le produit obtenu est le déhydrocitral. Il est obtenu avec un rendement de 54 %.

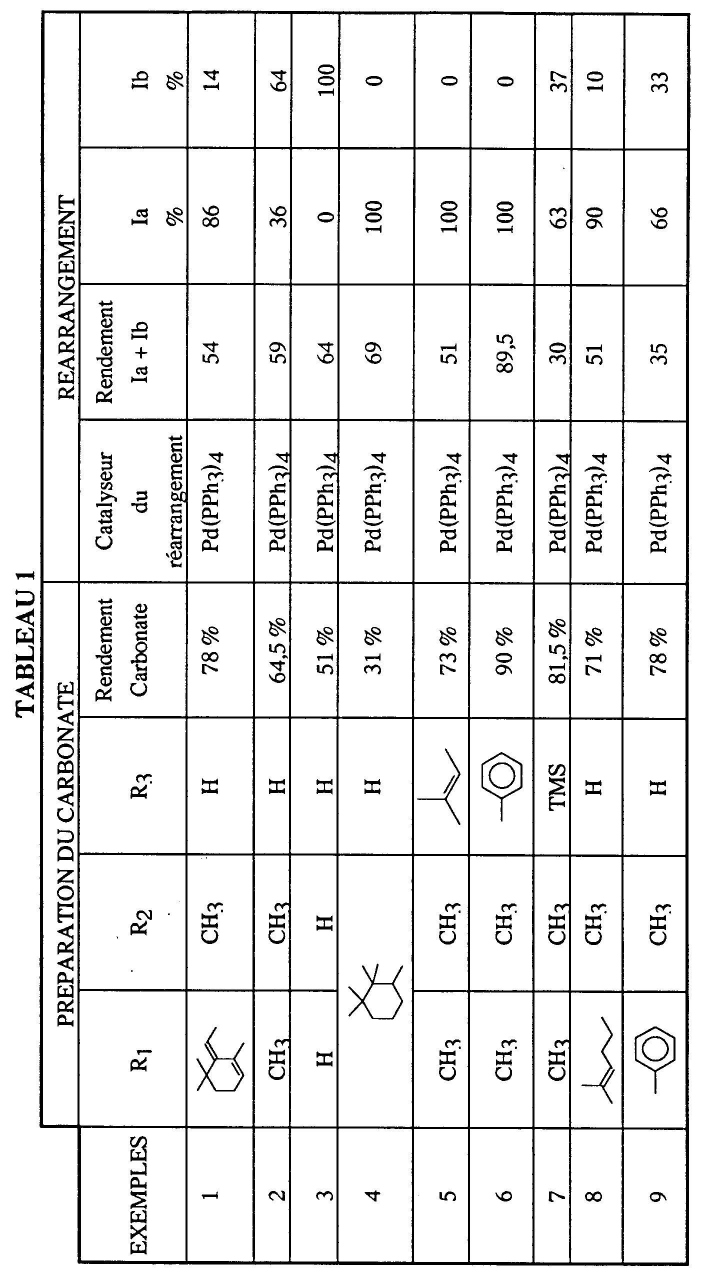

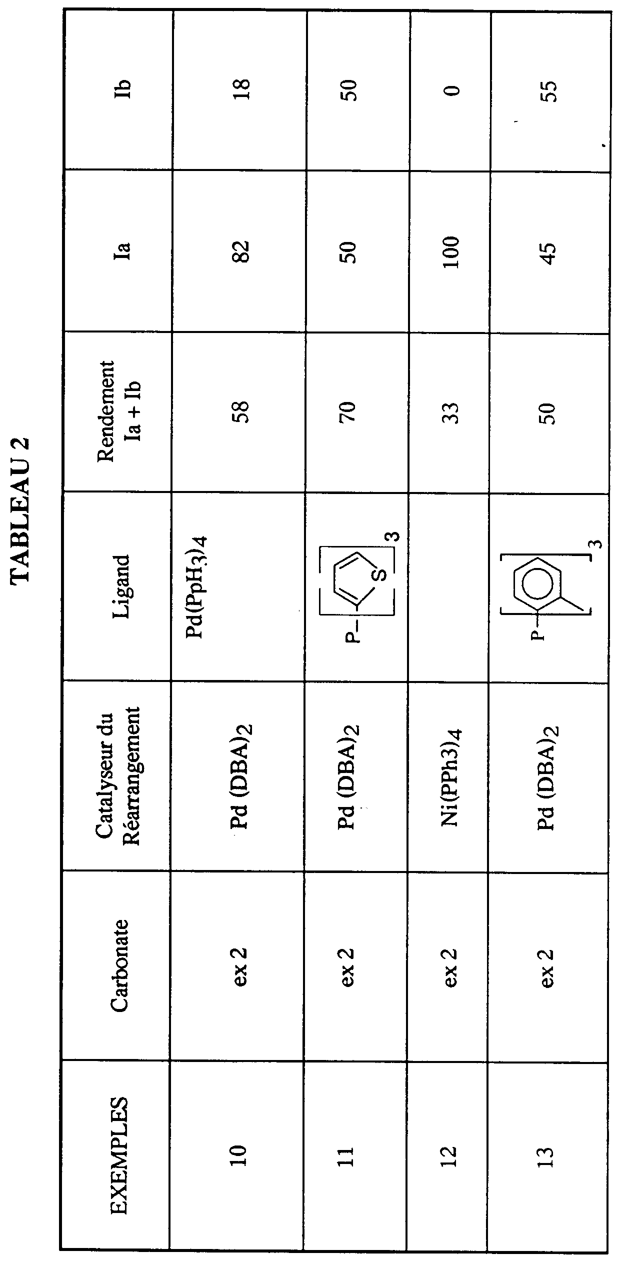

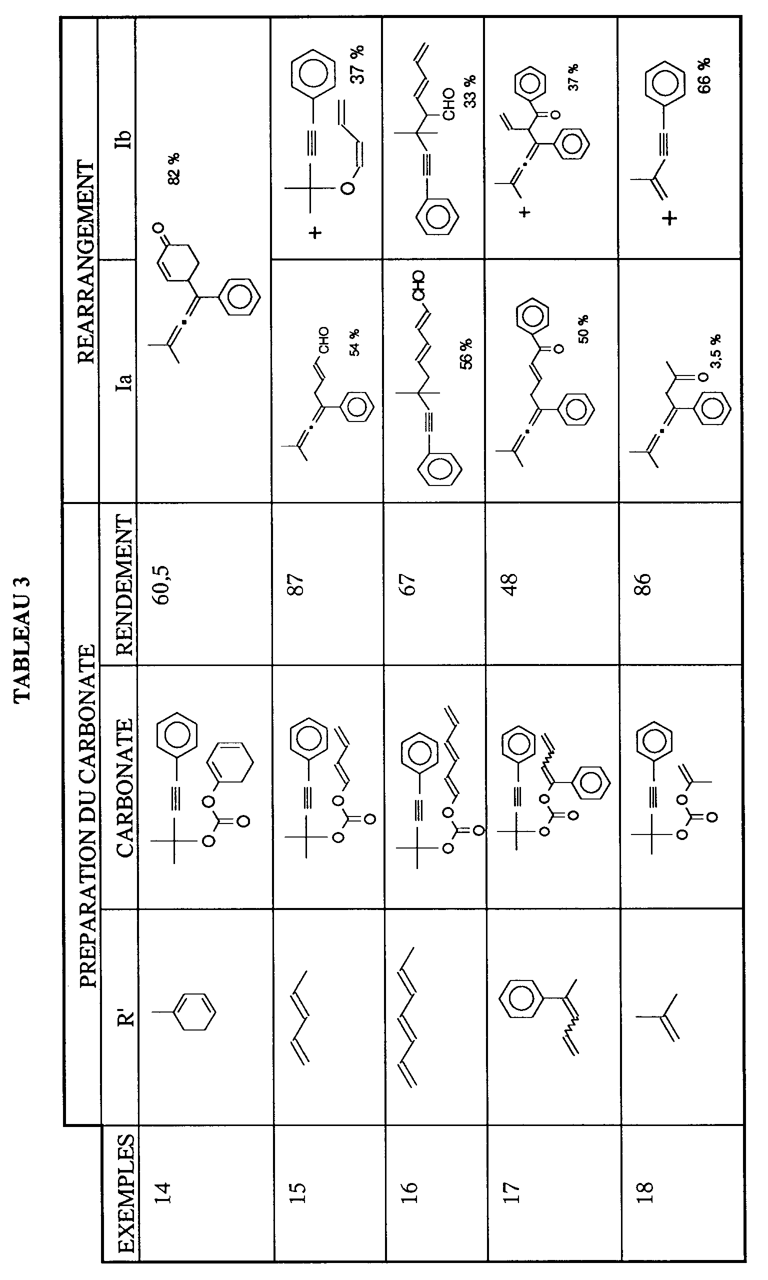

## Revendications

1. intermédiaires de préparation de la vitamine A et des caroténoïdes caractérisés en ce qu'ils répondent à la formule (I) suivante : 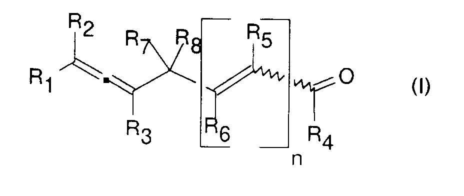 dans laquelle : - n est égal à 1 ou 2
- R₁, R₂ et R₃ représentent chacun indépendamment l'un de l'autre l'hydrogène, un motif alkyle contenant 1 à 4 atomes de carbone, un motif alkényle contenant 2 à 10 atomes de carbone, aryle, cycloalkyle, cycloalkényle, chaque motif alkyle et/ou alkényle pouvant être linéaire ou ramifié, deux groupes peuvent former ensemble un motif alkylidène contenant de 2 à 12 atomes de carbone,
- quand R₁, R₂ et R₆ représentent un groupe méthyle R₄, R₅, R₇ et R₈ représentent l'hydrogène et n est 1 R₃ peut représenter un groupe triméthylsilyl,
- R₄, R₅, R₆, R₇ et R₈ représentent chacun indépendamment l'un de l'autre l'hydrogène, un motif alkyle contenant 1 à 4 atomes de carbone ou alkényle contenant 2 à 10 atomes de carbone, chaque motif alkyle, alkényle étant linéaire, ramifié ou cyclique et aryle contenant 6 à 10 atomes de carbone; deux groupes peuvent former ensemble un motif alkylidene contenant 2 à 10 atomes de carbone.

2. intermédiaires selon la revendication 1 caractérisés en ce que R₄, R₅, R₇, R₈ représentent l'hydrogène et R₆ un groupe méthyle.

3. intermédiaires selon les revendications 1 et 2 caractérisés en ce que R1 représente le motif suivant : 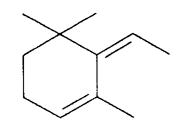 R2 un groupe méthyle et R3 l'hydrogène.

4. Procédé de préparation des intermédiaires de formule (I) caractérisé en ce qu'on fait réagir un intermédiaire de formule (II) suivante : 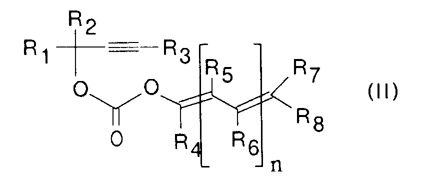 avec un catalyseur à base d'un métal de transition et éventuellement d'un ligand dans un solvant aprotique.

5. Procédé selon la revendication 4 caractérisé en ce que le métal est choisi parmi les métaux des colonnes VIII, I_{B} et II_{B} de la classification périodique.

6. Procédé selon la revendication 4 caractérisé en ce que le métal est choisi parmi le nickel et le palladium.

7. Procédé selon la revendication 4 caractérisé en ce que le ligand est une phosphine.

8. Procédé selon la revendication 4 caractérisé en ce que la quantité de catalyseur utilisée varie entre 1 et 20 % en mole par rapport au dérivé de formule (II).

9. Procédé de préparation du rétinal caractérisé en ce qu'on traite le dérivé de formule (I) dans lequel R₁ représente le groupe 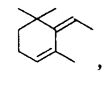 R₂ et R₆ un groupe méthyle, R₃, R₄, R₅, R₆ et R₇ l'hydrogène par l'acide bromhydrique.

10. Procédé de préparation du déhydrocitral caractérisé en ce qu'on traite le dérivé de formule (I) dans lequel R₁, R₂, R₆ représentent un groupe méthyle, R₃, R₄, R₅ et R₇ l'hydrogène par la carbonate de potassium.

11. Procédé de préparation du déhydrofarnesal caractérisé en ce qu'on traite le dérivé de formule (I) dans lequel R₁ représente le motif 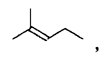 R₂ et R₆ représentent un groupe méthyle, R₃, R₄, R₅ et R₇ représentent l'hydrogène par l'acide bromhydrique.

## Claims

1. Intermediates for the preparation of vitamin A and carotenoids, characterized in that they correspond to the following formula (I): 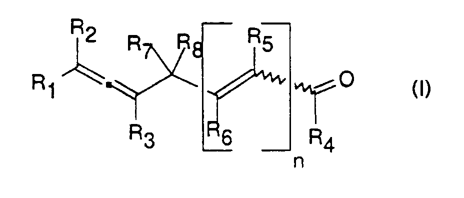 in which
- n is equal to 1 or 2
- R₁, R₂ and R₃, each independently represent hydrogen, an alkyl unit containing 1 to 4 carbon atoms, an alkenyl unit containing 2 to 10 carbon atoms or an aryl, cycloalkyl or cycloalkenyl unit, each alkyl and/or alkenyl unit possibly being linear or branched, two groups possibly forming together an alkylidene unit containing from 2 to 12 carbon atoms,
- when R₁, R₂, and R₆ represent a methyl group R₄, R₅, R₇ and R₈ represent hydrogen and n is 1, R₃ may represent a trimethylsilyl group,
- R₄, R₅, R₆, R₇ and R₈, each independently represent hydrogen, an alkyl unit containing 1 to 4 carbon atoms or an alkenyl unit containing 2 to 10 carbon atoms, each alkyl or alkenyl unit being linear, branched or cyclic, or an aryl unit containing 6 to 10 carbon atoms; two groups may together form an alkylidene unit containing 2 to 10 carbon atoms.

2. Intermediates according to claim 1, characterized in that R₄, R₅, R₇ and R₈ represent hydrogen and R₆ represents a methyl group.

3. Intermediates according to claims 1 and 2, characterized in that R₁ represents the following unit: 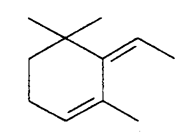 R₂ represents a methyl group and R₃ represents hydrogen.

4. Process for the preparation of the intermediates of formula (I), characterized in that an intermediate of the following formula (II): 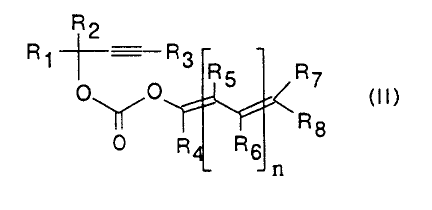 is reacted with a catalyst based on a transition metal and possibly a ligand, in an aprotic solvent.

5. Process according to claim 4, characterized in that the metal is chosen from the metals of colas VIII, I_{B} and II_{B} of the Periodic Classification.

6. Process according to claim 4, characterized in that the metal is chosen from nickel and palladium.

7. Process according to claim 4, characterized in that the ligand is a phosphine.

8. Process according to claim 4, characterized in that the amount of catalyst used ranges between 1 and 20 mol% relative to the derivative of formula (II).

9. Process for the preparation of retinal, characterized in that the derivative of formula (I) in which R₁ represents the group 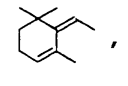 R₂ and R₆ represent a methyl group and R₃, R₄, R₅, R₆ and R₇ represent hydrogen is treated with hydrobromic acid.

10. Process for the preparation of dehydrocitral, characterized in that the derivative of formula (I) in which R₁, R₂ and R₆ represent a methyl group and R₃, R₄, R₅ and R₇ represent hydrogen is treated with potassium carbonate.

11. Process for the preparation of dehydrofernesal, characterized in that the derivative of formula (I) in which R₁ represents the unit 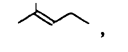 R₂ and R₆ represent a methyl group and R₃, R₄, R₅ and R₇ represent hydrogen is treated with hydrobromic acid.

## Patentansprüche

1. Zwischenprodukte zur Herstellung von Vitamin A und Carotenoiden, dadurch gekennzeichnet, daß sie der allgemeinen Formel (I) 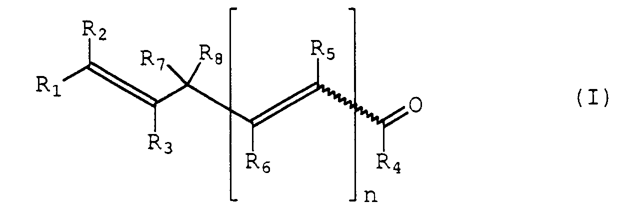 entsprechen, in der
- n gleich 1 oder 2 ist,
- R₁, R₂ und R₃, jeweils unabhängig voneinander, Wasserstoff, eine Gruppe Alkyl mit 1 bis 4 Kohlenstoffatomen, eine Gruppe Alkenyl mit 2 bis 10 Kohlenstoffatomen, Aryl, Cycloalkyl, Cycloalkenyl darstellen, wobei jede Gruppe Alkyl und/oder Alkenyl linear oder verzweigt sein kann und zwei Gruppen zusammen einen Rest Alkyliden mit 2 bis 12 Kohlenstoffatomen bilden können,
- wenn R₁, R₂ und R₆ eine Methylgruppe darstellen, R₄, R₅, R₇ und R₈ Wasserstoff bedeuten und n gleich 1 ist, und R₃ eine Gruppe Trimethylsilyl darstellen kann,
- R₄, R₅, R₆, R₇ und R₈, jeweils unabhängig voneinander, Wasserstoff, eine Gruppe Alkyl mit 1 bis 4 Kohlenstoffatomen oder Alkenyl mit 2 bis 10 Kohlenstoffatomen, wobei jede Gruppe Alkyl, Alkenyl linear, verzweigt oder cyclisch sein kann, und Aryl mit 6 bis 10 Kohlenstoffatomen darstellen, und zwei Gruppen zusammen eine Gruppe Alkyliden mit 2 bis 10 Kohlenstoffatomen bilden können.

2. Zwischenprodukte nach Anspruch 1, dadurch gekennzeichnet, daß R₄, R₅, R₇ und R₈ Wasserstoff darstellen und R₆ eine Methylgruppe ist.

3. Zwischenprodukte nach Anspruch 1 und 2, dadurch gekennzeichnet, daß R₁ die folgende Struktureinheit 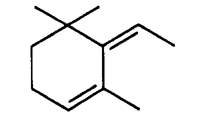 darstellt, R₂ eine Methylgruppe bedeutet und R₃ Wasserstoff ist.

4. Verfahren zur Herstellung der Zwischenprodukte der Formel (I), dadurch gekennzeichnet, daß man ein Zwischenprodukt der folgenden Formel (II) 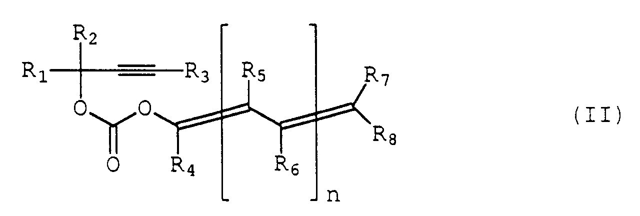 mit einem Katalysator auf der Basis eines Übergangsmetalles und gegebenenfalls eines Liganden in einem aprotischen Lösungsmittel zur Reaktion bringt.

5. Verfahren nach Anspruch 4, dadurch gekennzeichnet, daß das Metall unter den Metallen der Gruppen VIII, I_{B} und II_{B} des Periodensystems der Elemente ausgewählt wird.

6. Verfahren nach Anspruch 4, dadurch gekennzeichnet, daß das Metall unter Nickel und Palladium ausgewählt wird.

7. Verfahren nach Anspruch 4, dadurch gekennzeichnet, daß der Ligand ein Phosphin ist.

8. Verfahren nach Anspruch 4, dadurch gekennzeichnet, daß die Menge des verwendeten Katalysators zwischen 1 und 20 Mol-% variiert, bezogen auf das Derivat der Formel (II).

9. Verfahren zur Herstellung von Retinal, dadurch gekennzeichnet, daß man das Derivat der Formel (I), in der R₁ die Gruppe 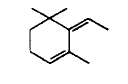 darstellt, R₂ und R₆ eine Methylgruppe bedeuten und R₃, R₄, R₅ und R₇ Wasserstoff sind, mit Bromwasserstoffsäure behandelt.

10. Verfahren zur Herstellung von Dehydrocitral, dadurch gekennzeichnet, daß man das Derivat der Formel (I), in der R₁, R₂, R₆ eine Methylgruppe darstellen, R₃, R₄, R₅ und R₇ Wasserstoff bedeuten, mit Kaliumcarbonat behandelt.

11. Verfahren zur Herstellung von Dehydrofarnesal, dadurch gekennzeichnet, daß man das Derivat der Formel (I), in der R₁ die Struktureinheit 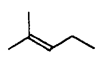 darstellt, R₂ und R₆ eine Methylgruppe sind, R₃, R₄, R₅ und R₇ Wasserstoff bedeuten, mit Bromwasserstoffsäure behandelt.
